# EUROPÄISCHE PATENTSCHRIFT

(11) **EP 2 861 126 B1**
(45) Veröffentlichungstag und Bekanntmachung des Hinweises auf die Patenterteilung: **20.01.2021**
(21) Anmeldenummer: 13741662.4
(22) Anmeldetag: 12.06.2013
(51) Int. Cl.: A61B 3/16, A61B 5/00, A61F 2/16

(54) **VORRICHTUNG ZUR OPTISCHEN DARSTELLUNG DES AUGENINNENDRUCKS**
DEVICE FOR OPTICALLY REPRESENTING INTRAOCULAR PRESSURE
DISPOSITIF DE REPRÉSENTATION VISUELLE DE LA PRESSION INTERNE DE L'OEIL

(30) Priorität: 13.06.2012 DE 102012105129
(43) Veröffentlichungstag der Anmeldung: 22.04.2015
(73) Patentinhaber: Christian-Albrechts-Universität zu Kiel, 24118 Kiel (DE)
(72) Erfinder: NAZIRIZADEH, Yousef, 65193 Wiesbaden (DE); GERKEN, Martina, 24116 Kiel (DE); KARROCK, Torben, 23715 Bosau (DE); ROIDER, Johann, 24105 Kiel (DE)
(74) Vertreter: Shalibeik, Hotan
(86) Internationale Anmeldenummer: PCT/DE2013/100214
(87) Internationale Veröffentlichungsnummer: WO 2013/185757

(56) Entgegenhaltungen:
- WO-A2-2004/062480
- WO-A2-2007/035356
- WO-A2-2010/100654
- WO-A2-2011/082314
- DE-A1-102005 041 271
- US-A1- 2009 299 216

## Beschreibung

Die Erfindung betrifft eine Vorrichtung nach Anspruch 1 zur optischen Darstellung des Augeninnendrucks. Im Rahmen von Kataraktoperationen werden in Deutschland jährlich etwa 650.000 Intraokularlinsen implantiert.

Der erhöhte Augeninnendruck ist in der Pathogenese einer der wesentlichen Gründe für Glaukome, die einen Verlust der Sehkraft oder des Gesichtsfeldes verursachen können. Die Zahl der Erblindungen durch Glaukom wird auf etwa 50.000 pro Jahr geschätzt. Rechtzeitige Messung des Augeninnendrucks und entsprechende medikamentöse Behandlung und rechtzeitige chirurgische Therapie könnten einer Erkrankung jedoch vorbeugen.

Die jetzigen Methoden sind jedoch durch eine Berührung mit dem Auge des Patienten unangenehm und auch nicht sehr aussagekräftig, da sie den Innendruck nur indirekt durch die Spannung der Augenaußenseite messen. In der Regel geschieht dies durch Applanationstonometrie, bei der eine Impression der Hornhaut durch Kontakt stattfindet. Da dies unangenehm ist, hat es zur Folge, dass die Augeninnendruckmessungen nur zeitlich punktuell durchgeführt werden, z.B. nur einige Male im Jahr. Dadurch kann nur ein Teil der Glaukompatienten überhaupt rechtzeitig erkannt werden.

Aus der WO 2004/062480 A2 ist ein Verfahren, Vorrichtungen und Systeme zur Messung von Druck und / oder zur quantitativen oder qualitativen Messung von Analyten in dem Auge oder anderen Körperteilen, bekannt.

Aus der WO 2007/035356 A2 ist eine in das menschliche Auge implantierbare Vorrichtung zur Behandlung von Glaukomen sowie ein diesbezügliches Verfahren bekannt.

Die DE 10 2005 041 271 A1 beschreibt ein Implantat zur Implantation in einen Kammerwasser enthaltenden Implantationsbereich eines Auges oder einen unmittelbar an Kammerwasser angrenzenden Implantationsbereich des Auges, das bei Bestrahlung des Auges und des in das Auge implantierten Implantats mit Anregungsstrahlung die Erzeugung von Raman-Strahlung durch Wechselwirkung der Anregungsstrahlung mit einem vorgegebenen Stoff im Kammerwasser des Auges wenigstens in einem Wechselwirkungsbereich in oder an dem Implantat verstärkt sowie eine Vorrichtung und ein Verfahren zur Ermittlung einer Konzentration wenigstens eines Stoffes im Kammerwasser und/oder im Gewebe des Auges.

Aus der WO 2010/100654 A2 ist eine Vorrichtung zur Überwachung des Augeninnendrucks bekannt, wobei der Patient diese selbst durchführen kann, indem Änderungen in Muster und Farbe des Gerätes erkannt werden.

Die WO 2011/082314 A2 beschreibt ein System, eine Vorrichtung sowie ein Verfahren zur Bestimmung des Augeninnendrucks, wobei die Bestimmung des Augeninnendrucks über einen Sensor erfolgt, wobei der Sensor mit einer oder mehreren Wellenlängen Licht beleuchtet wird und ein Detektor über das resultierende Licht Informationen über den Augeninnendruck erhält.

Aus der US 2009/0299216 A1 ist ein implantierbarer Drucksensor auf Polymerbasis bekannt, mit einem elektrischen LC-Tankschwingkreis für passive drahtlose Sensoren.

Eine sanftere Methode, bei der die Verformung der Hornhaut aufgrund eines Luftstoßes gemessen wird, liefert nur ungenaue Ergebnisse.

Bristow et al., "Polarization Conversion in the Reflectivity Properties of Photonic Crystal Waveguides", aus IEEE Journal of Quantum Electronics, Vol. 38, No. 7, July 2002 zeigt, dass der Polarisationszustand des Lichtes bei Reflexion an einem photonischen Kristall nahe/an der Resonanz geändert wird. Wie dort gezeigt, ändert sowohl ein 2D photonischer Kristall als auch ein 1D photonischer Kristall den Polarisationszustand des Lichtes an der Resonanz. Dieses geschieht sowohl in Reflexion als auch Transmission.

Nazirizadeh et al. "Optical characterization of photonic crystal slabs using orthogonally oriented polarization filters", 12 May 2008 / Vol. 16, No. 10 / OPTICS EXPRESS 7153 zeigt, dass bei Platzierung des photonischen Kristalls zwischen zwei Polarisationsfiltern, nur die Resonanzen aufgrund der Änderung des Polarisationszustandes passieren können.

Y. Nazirizadeh, U. Geyer, U. Lemmer and M. Gerken, "Spatially resolved optical characterization of photonic crystal slabs using direct evaluation of photonic modes," 2008 IEEE/LEOS International Conference on Optical MEMs and Nanophotonics, Freiburg, 2008, pp. 112-113, doi: 10.1109/OMEMS.2008.4607854 zeigt einen ortsabhängigen Farbeindruck für einen inhomogenen photonischen Kristall. Hier liegen herstellungsbedingt Änderungen in der optischen Mode vor. Hieraus kann erkannt werden, dass bei einem homogenen photonischen Kristall mit derselben Methode Änderungen an der Oberfläche detektiert werden können. Die Mode hat einen evaneszenten Anteil aus dem Wellenleiter heraus. Ändert sich der Brechungsindex an der Oberfläche, so verschiebt sich die Resonanz in der Wellenlänge. Dieses wiederum bewirkt, dass sich auch der wellenlängenabhängige Polarisationszustand ändert.

Die sich änderndeTransmissionsintensität bei zwei gekreuzten Polarisationsfiltern ist in Nazirizadeh et al. "Low-cost label-free biosensors using photonic crystals embedded between crossed polarizers", 30 August 2010 / Vol. 18, No. 18 / OPTICS EXPRESS 19120 gezeigt. Die Transmission kommt nur dadurch zustande, dass der photonische Kristall den Polarisationszustand an der Resonanz verändert.

Es wird daher angestrebt, dass der Patient mit Hilfe eines kleinen, vorteilhafterweise automatisierten Auslesegeräts selbstständig und berührungsfrei seinen Augeninnendruck bestimmen kann.

Es ist daher Aufgabe der Erfindung einen von außen auslesbaren Augeninnendrucksensor zur Auslesung durch entweder den Patienten selbst oder mit höherer Genauigkeit durch Apotheken oder Arztpersonal zur Verfügung zu stellen.
In einer einfachsten Variante wird eine rein visuelle Messung mit Hilfe einer speziellen Lupe durchgeführt, bei der der Patient die Farbkreisgröße des Sensors bestimmt.

Dabei wird das Verfahren zur optischen Darstellung des Augeninnendrucks mit einer erfindungsgemäß an einer Intraokularlinse im Auge implantierten Einrichtung arbeiten, die eine sich bei änderndem Augeninnendruck auswölbende Membran und eine Andruckfläche aufweist. Bei einer Erhöhung des Augeninnendrucks wird sich im Bereich des Kontakts der Membran mit der Fläche z.B. durch eine reversible chemische Reaktion oder durch andere optische Effekte die Polarisation für einen Spektralbereich einfallenden und reflektierten Lichts ändern.

Eine erfindungsgemäße Ausleseeinrichtung zur optischen Wiedergabe einer flächigen Abbildung des von dem photonischen Kristall reflektierten in der Polarisation geänderten Lichts, die einen Polarisationsfilter für das aufgestrahlte und das von dem photonischen Kristall reflektierte Licht aufweist, kann für die rein visuelle Messung mit Tageslicht arbeiten und beispielsweise aus einem Spiegel bestehen, mit dem der Patient sich Licht ins Auge lenkt. Vor dem Spiegel ist erfindungsgemäß ein Zirkularpolarisationsfilter vorzusehen, der das eingestrahlte und auch das rückgestreute Licht polarisiert. Die Betrachtung des Auges würde vom Patienten im Spiegel erfolgen, wobei er bei normalem Augeninnendruck keinen oder einen kleinen verfärbten Bereich erkennen würde.

Erfindungsgemäß wird zur Polarisationsänderung vorgeschlagen, die Membran auf eine nanostrukturierte Oberfläche eines benachbart in der Intraokularlinse befestigten für den Träger optisch transparenten photonischen Kristalls hin auslenkbar zu gestalten, so dass sie bei erhöhtem Augeninnendruck mit einem flächigen Bereich an der nanostrukturierten Oberfläche anliegt. Zur besseren Ausgestaltung einer Kennlinie kann diese Oberfläche noch ebenfalls gewölbt ausgebildet sein. Es ist auch denkbar eine Mehrzahl von Kontaktpunkten oder Ringen auf diese Oberfläche einzuprägen, um der Darstellung einen stufenweisen Verlauf zu geben.

Schließlich wird auch ein mit definierten bekannten geometrischen Abmessungen versehener Referenzmaßstab auf der Andruckfläche möglich sein oder es kann ein ohnehin vorhandener Barcode oder ID-Nummer so nahe an den Bereich des Kontakts zwischen Membran und Oberfläche gerückt werden, dass insbesondere bei einer Kamera- und Software-Auswertung die Fläche des Referenzmaßstabs im Bild ist.

Bei einer automatisierten Ausleseeinrichtung wird vorgeschlagen, mit definierter Beleuchtung zu arbeiten und eine Kamera als Teil der Ausleseeinrichtung vorzusehen, wobei ein speicherbares Bild erzeugt wird. Bei dieser Ausgestaltung können zwei longitudinal polarisierende Filter vorgesehen werden, wobei derjenige im Einstrahlstrahlengang und derjenige für das reflektierte Licht um 90° gekreuzt sind, zur Darstellung des Bereichs des Flächenkontakts als Differenzbild.

Das erfasste Kamerabild kann dann mit Rechnermitteln anhand einer im Bild enthaltenen vorbekannten Referenzgröße für die Bestimmung eines Zahlenwerts des Augeninnendrucks aus der Größe der erfassten Kontaktfläche bearbeitet werden.

Erfindungsgemäß befindet sich die nanostrukturierte Oberfläche innerhalb einer Referenzdruckkammer, die durch eine Membran abgeschlossen wird, in der Intraokularlinse befindet, deren Innendruck so bemessen ist, dass bei normalem Augeninnendruck kein oder nur minimaler Kontakt zwischen Membran und Oberfläche entsteht.

Weitere Merkmale und Vorteile der Erfindung ergeben sich aus der nachfolgenden Beschreibung eines bevorzugten Ausführungsbeispiels. Dabei zeigt:
- **Fig. 1**: eine schematische Darstellung einer Kunstlinse mit einem integriertem Augeninnendrucksensor am Rand und
- **Fig. 2**: einen Messaufbau zum Auslesen des Druckwertes in Form einer je nach Druck größeren oder kleineren sichtbaren Fläche.

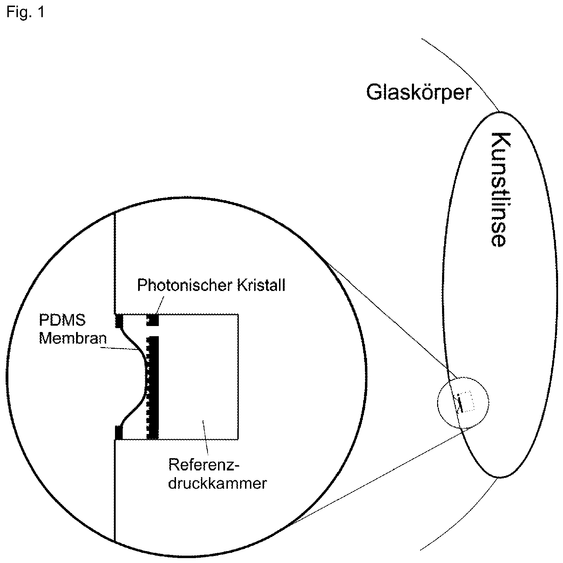

Wie in **Fig. 1** gezeigt, besteht der Augeninnendrucksensor aus einer beweglichen Membran, einer periodisch nanostrukturierten Oberfläche (photonischer Kristall) und einer Referenzdruckkammer. Der Referenzdruck wird dabei so voreingestellt, dass bei dem niedrigsten Augeninnendruckwert die Membran gerade die photonische Kristalloberfläche berührt. Es ist denkbar, für verschiedene mögliche Augeninnendrücke einen Satz von jeweils mit einem anderen Referenzdruck versehenen Kunstlinsen vorzusehen, so dass bei der Implantation eine für den jeweiligen Patienten geeignete Linse eingesetzt werden kann.

Bei einer Druckerhöhung im Auge wird die Membran dann in einem größeren Bereich an den photonischen Kristall gedrückt. Das Reflexionsspektrum unterscheidet sich örtlich, je nachdem, ob die Membran an den photonischen Kristall angedrückt ist oder nicht, da die angedrückte Membran zu einer Brechungsindexänderung an der Oberfläche des photonischen Kristalls führt.

Beim Auslesen des Sensors mit einer breitbandigen Lichtquelle (sichtbares Licht, Tageslicht) ergibt sich dadurch ein anderer Farbeindruck in Bereichen, in denen die Membran angedrückt ist. Zum Beispiel kann der Bereich mit angedrückter Membran rot erscheinen, während die übrige Oberfläche gelb erscheint. Ändert sich der Druck, so ändert sich die Größe z.B. eines roten Kreises und gleichzeitig nimmt dieser Bereich der Einfärbung mehr der übrigen Fläche ein.

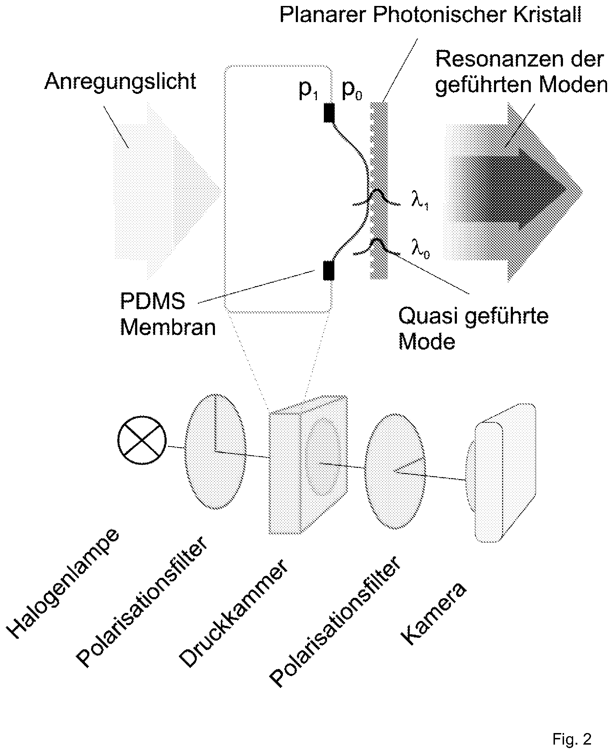

**Fig. 2** zeigt einen Messaufbau zum Auslesen des Druckwertes in Form einer je nach Druck größeren oder kleineren sichtbaren Fläche.

## Patentansprüche

1. Vorrichtung zur optischen Darstellung des Augeninnendrucks umfassend eine an einer Intraokularlinse im Auge implantierten Einrichtung mit einer Referenzdruckkammer, eine Ausleseeinrichtung, und einen Zirkularpolfilter,
wobei die implantierte Einrichtung
- eine sich bei änderndem Augeninnendruck auswölbende Membran und einen photonischen Kristall als Andruckfläche für die Membran enthält;
und wobei
- die Membran auf eine nanostrukturierte Oberfläche des benachbart in der Intraokularlinse befestigten für den Träger optisch transparenten photonischen Kristalls hin auslenkbar und bei erhöhtem Augeninnendruck mit einem flächigen Bereich an der Andruckfläche anliegend vorgesehen ist;
und wobei
- die Andruckfläche im Bereich des Kontakts mit der Membran für aufgestrahltes und reflektiertes Licht eines Spektralbereiches die Polarisation ändert;
und wobei
- die Ausleseeinrichtung zur optischen Wiedergabe einer flächigen Abbildung des von dem photonischen Kristall reflektierten in der Polarisation geänderten Lichts eingerichtet ist und einen Polarisationsfilter für aufgestrahltes und das von dem photonischen Kristall reflektierte Licht aufweist; und wobei
- die nanostrukturierte Oberfläche sich innerhalb der Referenzdruckkammer, die durch die Membran abgeschlossen wird, in der Intraokularlinse befindet,
und wobei
- der Innendruck der Referenzdruckkammer so bemessen ist, dass bei normalem Augeninnendruck kein oder nur minimaler Kontakt zwischen Membran und Oberfläche entsteht,
und wobei
- der Zirkularpolfilter für eingestrahltes sichtbares Licht und im Rückstrahl für das beobachtete reflektierte Streulicht eingesetzt wird.

2. Vorrichtung nach Anspruch 1,
**dadurch gekennzeichnet, dass**
ein mit definierten bekannten geometrischen Abmessungen versehener Referenzmaßstab auf der Andruckfläche vorgesehen ist.

3. Vorrichtung nach einem der vorangehenden Ansprüche,
**dadurch gekennzeichnet, dass** die Ausleseeinrichtung mit definierter Beleuchtung und eine Kamera als Teil der Ausleseeinrichtung vorgesehen sind, wobei ein speicherbares Bild erzeugt wird.

4. Vorrichtung nach Anspruch 3,
**dadurch gekennzeichnet, dass**
zwei longitudinal polarisierende Filter vorgesehen sind, wobei derjenige im Einstrahlstrahlengang und derjenige für das reflektierte Licht um 90° gekreuzt sind, zur Darstellung des Bereichs des Flächenkontakts als Differenzbild.

5. Vorrichtung nach Anspruch 3 oder 4 mit einem Rechner, der dazu eingerichtet ist, das Kamerabild anhand einer im Bild enthaltenen vorbekannten Referenzgröße zur Bestimmung eines Zahlenwerts des Augeninnendrucks aus der Größe der erfassten Kontaktfläche zu bearbeiten.

## Claims

1. Apparatus for optical representation of the intraocular pressure comprising a device on an intraocular lens implanted in the eye with a reference pressure chamber, a read-out device, and a circular polarizing filter, wherein the implanted device
- contains a membrane that bulges out as the intraocular pressure changes and a photonic crystal as a contact surface for the membrane;
and wherein
- the membrane can be deflected onto a nanostructured surface of the photonic crystal which is attached adjacent to it in the intraocular lens and which is optically transparent for the user, and in the case of increased intraocular pressure is intended to contact the contact surface with a flat area;
and wherein
- the contact surface in the area of contact with the membrane changes polarisation for radiated and reflected light of a spectral range;
and wherein
- the read-out device is set up for the optical representation of a planar image of the polarisation-altered light reflected by the photonic crystal and comprises a polarisation filter for light which is irradiated and light reflected by the photonic crystal;
and wherein
- the nanostructured surface is located inside the reference pressure chamber, which is enclosed by the membrane, in the intraocular lens and wherein
- the internal pressure of the reference pressure chamber is dimensioned in such a way that, at normal intraocular pressure, there is no or only minimal contact between membrane and surface
and wherein
- the circular polarising filter is used for irradiated visible light and in the return beam for the observed reflected scattered light.

2. Apparatus according to claim 1,
**characterized in that**
a reference scale provided with defined known geometric dimensions is provided on the contact surface.

3. Apparatus according to any of the preceding claims,
**characterized in that**
the read-out device with defined illumination and a camera are provided as part of the read-out device, whereby a storable image is generated.

4. Apparatus according to claim 3,
**characterized in that**
two longitudinally polarizing filters are provided, the one in the incident beam path and the one for the reflected light being crossed by 90°, for representing the area of surface contact as a differential image.

5. Apparatus according to claim 3 or 4 comprising a computer, arranged to process the camera image using a preknown reference value contained in the image to determine a numerical value of intraocular pressure from the size of the detected contact area.

## Revendications

1. Dispositif de représentation visuelle de la pression intraoculaire comprenant un dispositif implanté sur une lentille intraoculaire dans l'oeil avec une chambre de pression de référence, un dispositif de lecture et un filtre polarisant circulaire, dans lequel le dispositif implanté
- contient une membrane qui se déforme lorsque la pression intraoculaire change et un cristal photonique qui sert de surface de contact pour la membrane;
et dans lequel
- la membrane peut être déviée vers une surface nanostructurée du cristal photonique qui est fixée à côté d'elle dans la lentille intraoculaire et est optiquement transparente pour le patient, et dans le cas d'une pression intraoculaire accrue, est munie d'une zone plane reposant contre la surface de contact;
et dans lequel
- la surface de contact dans la zone de contact avec le membrane change de polarisation pour la lumière rayonnée et réfléchie d'un domaine spectral; et dans lequel
- le dispositif de lecture est conçu pour la reproduction optique d'une image plane de la lumière à polarisation modifiée réfléchie par le cristal photonique et possède un filtre de polarisation pour la lumière qui est irradiée et la lumière qui est réfléchie par le cristal photonique;
et dans lequel
- la surface nanostructurée est située à l'intérieur de la chambre de pression de référence, fermée par la membrane, dans la lentille intraoculaire
et dans lequel
- la pression interne de la chambre de pression de référence est telle qu'à une pression intraoculaire normale, il n'y a pas ou peu de contact entre la membrane et la surface,
et dans lequel
- le filtre polarisant circulaire est utilisé pour la lumière visible incidente et dans le retour pour la lumière parasite réfléchie observée.

2. Dispositif selon la revendication 1,
**caractérisé en ce que**
une graduation de référence avec des dimensions géométriques connues définies est fournie sur la surface de contact.

3. Dispositif selon l'une des revendications précédentes,
**caractérisé en ce que**
le dispositif de lecture avec un illumination définie et une caméra sont fournis en tant que partie du dispositif de lecture, une image mémorisable étant générée.

4. Dispositif selon la revendication 3,
**caractérisé en ce que**
deux filtres polarisants longitudinaux sont prévus, dans lesquels celui du trajet du faisceau entrant et celui de la lumière réfléchie sont croisés à 90°, pour représenter la zone de contact de surface comme une image différentielle.

5. Dispositif selon la revendication 3 ou 4 avec un calculateur qui est configuré pour traiter l'image de la caméra en utilisant une valeur de référence préalablement connue contenue dans l'image pour déterminer une valeur numérique de la pression intraoculaire à partir de la taille de la zone de contact détectée.
